# EUROPÄISCHE PATENTSCHRIFT

(11) **EP 3 006 061 B1**
(45) Veröffentlichungstag und Bekanntmachung des Hinweises auf die Patenterteilung: **01.02.2017**
(21) Anmeldenummer: 14188393.4
(22) Anmeldetag: 10.10.2014
(51) Int. Cl.: A61M 3/02, A61F 9/007

(54) **Verfahren zur Steuerung des Irrigationsdrucks**
Method for controlling irrigation pressure
Procédé de commande de la pression d'irrigation

(43) Veröffentlichungstag der Anmeldung: 13.04.2016
(73) Patentinhaber: EOS GmbH, 52249 Eschweiler (DE)
(72) Erfinder: Klomp, Manfred, 4191AA Geldermalsen (NL)
(74) Vertreter: Schwarz & Partner

(56) Entgegenhaltungen:
- EP-A1- 0 717 970
- WO-A1-2009/017921
- WO-A1-2011/105909
- US-A- 1 956 006
- US-A- 5 032 111

## Beschreibung

Die Erfindung betrifft eine Vorrichtung zur Verwendung in der Augenchirurgie, umfassend ein Gasdruckgerät zur Bereitstellung eines variablen Ausgangsdrucks, ein Fluidbehältnis, zur Bereitstellung eines Fluids zum Spülen eines Auges, wobei das Fluidbehältnis an einer in Betriebslage unteren Seite, mit einem chirurgischen Handstück verbunden ist, um das Fluid mit einem vom Operateur vorgebbaren Irrigationsdruck in das Auge abzugeben.

Das Dokument US 5 032 111 offenbart so eine Vorrichtung, die Teil eines Augenoperationsgeräts ist und mit der die Linse eines Auges eines Patienten entfernt werden kann. Bei der Operation wird vorerst vom Operateur ein Schnitt an dem Auge gesetzt, über den die in kleine Stücke zerteilte alte Linse entfernt wird. Um das Entfernen der kleinen Stücke der alten Linse zu ermöglichen und um zu verhindern, dass das von der alten Linse ursprünglich eingenommene Volumen im Auge bei der Operation kollabiert, muss von dem chirurgischen Handstück das Fluid mit einem Irrigationsdruck in das operierte Auge abgegeben werden. Ein zu hoher Irrigationsdruck würde das Auge bleibend schädigen und bei einem zu geringen Irrigationsdruck ist die Gefahr des Kollabierens des Auges gegeben, weshalb der Operateur den geeigneten Irrigationsdruck einstellen können muss. Hierfür ist zwischen dem Fluidbehältnis und dem chirurgischen Handstück eine flexible Leitung ausgebildet. Das Fluidbehältnis ist des Weiteren über eine weitere Leitung mit dem Gasdruckgerät verbunden, diese ist dazu ausgebildet um den Irrigationsdruck über das Gasdruckgerät zu steuern. Für diese Steuerung kann der Ausgangsdruck am Steuergerät gesenkt oder erhöht werden, diese bidirektionale Druckregelung des Ausgangsdrucks erfordert, dass die Leitung zwischen Gasdruckgerät und dem Fluidbehältnis nur Gas führt. Hierfür umfasst das Leitungsende das im Inneren des Fluidbehältnisses liegt ein langes Rohr, das in der Gasphase des Fluidbehältnisses endet. Mit dem Rohr wird zum Verbinden des Fluidbehältnisses mit dem Gasdruckgerät, die Versiegelung des Fluidbehältnisses durchbrochen.

Es hat sich als Nachteil erwiesen, dass die erforderliche Länge dieses Rohrs das Herstellen der Verbindung und das Durchbrechen der Versiegelung nachteilig erschwert. Ein kürzeres Rohr würde die Handhabung zwar erleichtern, aber in die Leitung zwischen Gasdruckgerät und Fluidbehältnis könnte auch Fluid eindringen. Dadurch könnte das Gasdruckgerät zu Schaden kommen, beziehungsweise würde die bidirektionale Druckregelung zwischen Gasdruckgerät und Fluidbehältnis und somit die Steuerung des Irrigationsdrucks unmöglich sein.

Der Erfindung liegt die Aufgabe zu Grunde eine Vorrichtung zur Verwendung in der Augenchirurgie zu schaffen, die, unter der Vorrausetzung, dass die bidirektionale Druckregelung bestehen bleibt, das Verbinden des Fluidbehältnisses mit dem Gasdruckgerät für den Benutzer erleichtert.

Erfindungsgemäß wird diese Aufgabestellung dadurch gelöst, dass das Fluidbehältnis an einer in Betriebslage oberen Seite, mit einem Anschlusselement verbunden ist, wobei das Fluidbehältnis über einen Dorn des Anschlusselements, an einer unteren Seite eines Gasphasenbehältnis, mit dem Gasphasenbehältnis in Verbindung steht, und wobei das Gasphasenbehältnis und / oder das Fluidbehältnis eine Gasphase aufweist und die Gasphase über das Anschlusselement oder einer Fluidbehältnisleitung mit dem Gasdruckgerät in Verbindung steht, wodurch über den variablen Ausgangsdruck P_{A} des Gasdruckgeräts der Irrigationsdruck P_{I} des chirurgischen Handstücks regelbar ist.

Hierdurch ist der Vorteil erhalten, dass die Verbindung zwischen der Gasphase und dem Gasdruckgerät ohne unhandliche Komponenten hergestellt werden kann und die bidirektionale Druckregelung zwischen dem Fluidbehältnis und dem Gasdruckgerät bestehen bleibt. Hierfür wird die Verbindung zwischen dem Gasdruckgerät und dem Fluidbehältnis um das Gasphasenbehältnis erweitert. Mit dem Gasphasenbehältnis wird das Fluid für die Operation bereitgestellt. Während der Operationsvorbereitung wird das Fluid in das Fluidbehältnis abgelassen und zwar soweit, dass entweder der Endteil der Leitung zwischen Gasdruckgerät und Gasphasenbehältnis, im Gasphasenbehältnis in die Gasphase über den Fluidspiegel ragt, oder dass, wie im bevorzugten Ausführungsbeispiel, die Gasphase im Fluidbehältnis über die Fluidbehältnisleitung mit dem Gasdruckgerät in Verbindung steht. Dadurch kann vorteilhafterweise der Endteil der Leitung aus einem kurzen Rohr oder einem Dorn gebildet sein und über die entsprechende gasführende Leitung kann der Irrigationsdruck durch den variablen Ausgangsdruck des Gasdruckgeräts gesteuert werden. Durch Verringerung oder Erhöhung des Ausgangsdrucks wird auch der Irrigationsdruck abgesenkt oder angehoben.

Mit dem darauf folgenden Entlüften der Leitung zwischen dem Fluidbehältnis und dem chirurgischen Handstück mittels dem Fluid, wird das Fluid am chirurgischen Handstück bereitgestellt.

Weitere vorteilhafte Ausgestaltungen der erfindungsgemäßen Vorrichtung werden im Folgenden anhand der Figuren näher erläutert.
Figur 1 zeigt einen schematischen Aufbau einer erfindungsgemäßen Vorrichtung.
Figur 2 zeigt eine Teilansicht des Anschlusselements der erfindungsgemäßen Vorrichtung aus Figur 1, wobei das Anschlusselement geschnitten dargestellt ist.
Figur 3 zeigt eine Teilansicht eines Anschlusselements mit einem Abdeckmittel einer erfindungsgemäßen Vorrichtung, wobei das Anschlusselement geschnitten dargestellt ist.
Figur 4 zeigt eine Teilansicht eines Anschlusselements mit einem Dreiwegehahn einer erfindungsgemäßen Vorrichtung, wobei das Anschlusselement geschnitten dargestellt ist.
Figur 5 zeigt eine Teilansicht eines Verbindungsaufbaus zwischen einem Gasphasenbehältnis und einem Fluidbehältnis mit einer Fluidbehältnisleitung eines bevorzugten Ausführungsbeispiels einer erfindungsgemäßen Vorrichtung.
Figur 6 zeigt eine Teilansicht eines Verbindungsaufbaus zwischen einem Gasphasenbehältnis und einem Fluidbehältnis einer erfindungsgemäßen Vorrichtung, wobei ein Anschlusselement einen Dreiwegehahn aufweist.
Figur 7 zeigt eine Teilansicht eines Verbindungsaufbaus zwischen einem Gasphasenbehältnis und einem Fluidbehältnis einer erfindungsgemäßen Vorrichtung, wobei ein Anschlusselement eine Fluidabscheidevorrichtung aufweist.

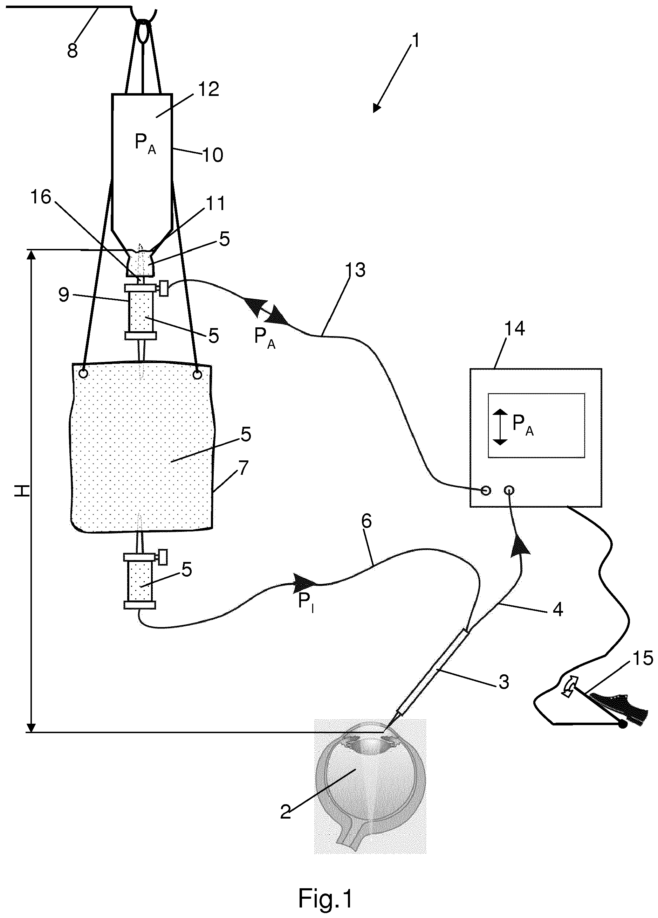

Figur 1 zeigt einen schematischen Aufbau einer Vorrichtung 1 zur Verwendung in der Augenchirurgie, mit einem zu behandelnden Auge 2, in das über eine operative Öffnung ein chirurgisches Handstück 3 eingeführt ist. Das chirurgische Handstück 3 weist eine Schneidvorrichtung auf, die sich am Ende des chirurgischen Handstücks 3 im Inneren des Auges 2 befindet. Diese Schneidvorrichtung dient zum Abtrennen von Augengewebe, das über eine im chirurgischen Handstück 3 liegende Leitung, die zu einer Leitung 4 führt, aus dem Auge 2 gespült wird. Für diesen Spülvorgang wird im Auge 2 über das chirurgische Handstück 3 ein Fluid 5 mit einem Irrigationsdruck P_{I} bereitgestellt. Hierfür ist das chirurgische Handstück 3 über eine Leitung 6 mit einem Fluidbehältnis 7 verbunden, wobei das Fluidbehältnis 7 an einem Infusionshalter 8 aufgehängt und mit dem Fluid 5 befüllt ist. An einem oberen Ende ist das Fluidbehältnis 7 über ein Anschlusselement 9 mit einem Gasphasenbehältnis 10, das ebenfalls am Infusionshalter 8 aufgehängt ist, verbunden. Ein Fluidspiegel 11 ist die Grenze zwischen dem Fluid 5 und einer Gasphase 12. Die Gasphase 12 steht, über das Anschlusselement 9 und über eine Leitung 13, in Verbindung mit einem Gasdruckgerät 14. Das Gasdruckgerät 14 besteht aus einer Gaspumpe und einer Regelung zum Erzeugen eines variablen Ausgangsdruck P_{A}. Die Regelung für den gewünschten Ausgangsdruck P_{A} wird über ein Fußpedal 15, welches vom Operateur bedient wird, angesteuert. Über die Leitung 13 herrscht in der Gasphase 12 ebenfalls der eingestellte Ausgangsdruck P_{A}. Der Irrigationsdruck P_{I} ergibt sich aus der Summe des hydrostatischen Drucks über eine Höhe H und des einstellbaren Ausgangsdrucks P_{A}. Über die fußbetätigte Regelung des Ausgangsdrucks P_{A} erfolgt somit auch die Steuerung des erforderten Irrigationsdruck P_{I}.

Es kann erwähnt werden, dass das Gasphasenbehältnis 10 durch eine handelsübliche Flasche, die mit einer Kochsalzlösung gefüllt ist, gebildet ist. Diese Flasche ist ein gängiges Produkt für den medizinischen Bereich. Des Weiteren kann das Anschlusselement einen Dorn eines handelsüblichen Infusionsgeräts aufweisen, wobei unter handelsübliches Infusionsgerät zum Beispiel das Produkt Intrafix® SafeSet der Firma Braun verstanden wird. Hiermit ist der Vorteil erhalten, dass durch den Einsatz von standardisierten, handelsüblichen Produkten einerseits die Handhabung für den Benutzer wesentlich erleichtert wird, andererseits können so die Kosten der Vorrichtung verringert werden.

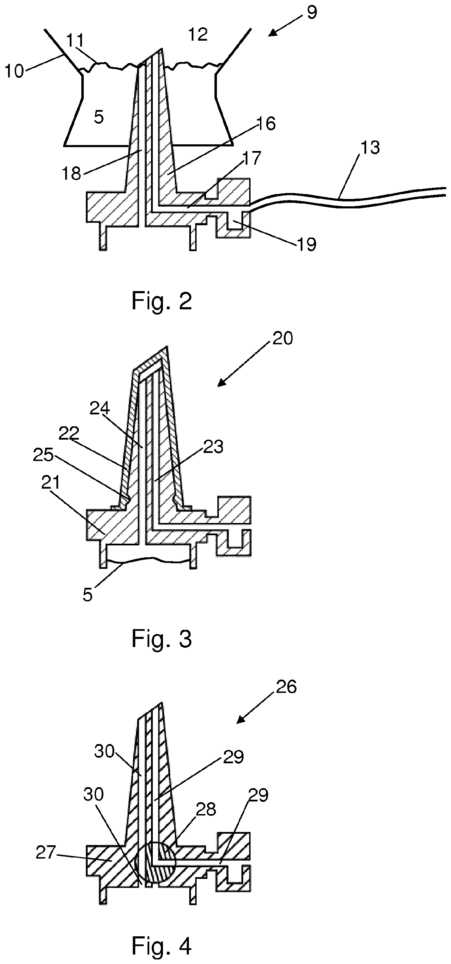

Figur 2 zeigt einen oberen Abschnitt des Anschlusselements 9 der erfindungsgemäßen Vorrichtung 1 nach Figur 1. Das Anschlusselement 9 umfasst einen Dorn 16, in dem ein erster Kanal 17 und ein zweiter Kanal 18 vorgesehen ist. Der erste Kanal 17 und die Leitung 13 verbinden das Gasphasenbehältnis 10 mit dem Gasdruckgerät 14. Am ersten Kanal 17 ist eine Fluidabscheidevorrichtung 19 ausgebildet, die dazu ausgebildet ist das Gasdruckgerät 14 vor ungewollten Fluideintritten zu schützen. Ein von dem Gasphasenbehältnis 10 bei reduziertem Ausgangsdruck P_{A} gegebenenfalls in den ersten Kanal 17 eintretendes Fluid 5 wird aufgrund der Schwerkraft in der Fluidabscheidevorrichtung 19 gesammelt, wohingegen das Gas mit dem Ausgangsdruck P_{A} von dem Gasdruckgerät 14 die Fluidabscheidevorrichtung 19 ungehindert passieren kann. Der zweite Kanal 18 verbindet das Gasphasenbehältnis 10 mit dem Fluidbehältnis 7. Bei der Vorrichtung 1 gemäß Figur 1 und Figur 2 erfordert die bidirektionale Druckregelung einen Schutz vor Fluideintritten, der ebenfalls eine bidirektionale Gasführung ermöglicht. Somit ist der Einsatz eines Einwegeventils, das Fluideintritte in Leitung 13 verhindert, in diesem Ausführungsbeispiel nicht zielführend.

Figur 1 und Figur 2 zeigen die Vorrichtung 1 nach durchgeführter Operationsvorbereitung, die im Wesentlichen aus folgenden Verfahrensschritten besteht. Das versiegelte Gasphasenbehältnis 10 ist mit dem Fluid 5 gefüllt und wird am Infusionshalter 8 aufgehängt. Mit dem Anschlusselement 9 werden bei der Operationsvorbereitung das Gasdruckgerät 14 über den ersten Kanal 17 mit dem Gasphasenbehältnis 10 und das Gasphasenbehältnis 10 über den zweiten Kanal 18 mit dem Fluidbehältnis 7 verbunden. Die Verbindung wird mittels Dorn 16 hergestellt, mit dem die nicht dargestellte Versiegelung des Gasphasenbehältnisses 10 durchbrochen wird. Zur Steuerung des Irrigationsdrucks P_{I} während der Operation muss der erste Kanal 17 in der Gasphase 12 enden. Hierfür wird gemäß dem erfindungsgemäßen Verfahren mit dem Ablassen des Fluids 5 vom Gasphasenbehältnis 10 in das Fluidbehältnis 7 begonnen. Beim Ablassen wird das Fluidbehältnis 7 über den zweiten Kanal 18 mit dem Fluid 5 gefüllt, wobei der im Gasphasenbehältnis 10 entstehende Unterdruck über den ersten gasführenden Kanal 17 ausgeglichen wird. Nach vollständigem Ablassen des Fluids 5 in das Fluidbehältnis 7 ist das Gasdruckgerät 14 direkt mit der Gasphase 12 verbunden und das Fluidbehältnis 7 kann mit dem Ausgangsdruck P_{A} beaufschlagt werden. Anschließend erfolgt die Bereitstellung des Fluids 5 am chirurgischen Handstück 3 mit dem Irrigationsdruck P_{I}. Durch die bidirektionale Regelung des Ausgangsdrucks P_{A} kann der Irrigationsdruck P_{I} je nach Vorgabe des Operateurs vorteilhafterweise besonders rasch erhöht oder gesenkt werden.

Figur 3 zeigt einen oberen Abschnitt eines Anschlusselements 20 einer erfindungsgemäßen Vorrichtung, das anstatt des Anschlusselements 9 auch in der Vorrichtung 1 verwendet werden kann. Das Anschlusselement 20 umfasst einen Dorn 21 und ein Abdeckmittel 22. Innerhalb des Anschlusselements 20 erstrecken sich ein erster Kanal 23 und ein zweiter Kanal 24. Ein gasdichter, elastischer Verriegelungsverschluss 25 ist am Anschlusselement 20 und am Abdeckmittel 22 dazu ausgebildet, um den ersten Kanal 23 mit dem zweiten Kanal 24 gasdicht zu verbinden.

Die Operationsvorbereitung für das Ausführungsbeispiel gemäß Figur 1 mit dem Anschlusselement 20 gemäß Figur 3 entspricht, bis auf einen zusätzlichen Verfahrenschritt, der Beschreibung zu Figur 1 und Figur 2. Der zusätzliche Verfahrensschritt ist dadurch gegeben, dass nach dem Ablassen des Fluids 5 von dem Gasphasenbehältnis 10 in das Fluidbehältnis 7 das Gasphasenbehältnis 10 vom Anschlusselement 20 getrennt und danach das Anschlusselement 20 mit dem Abdeckmittel 22 abgedeckt wird. Durch das Abtrennen des Gasphasenbehältnisses 10 wird das Volumen der Gasphase 12 verringert, wodurch für die Regelung auch weniger Gas, je nach eingestelltem Ausgangsdruck P_{A}, komprimiert oder expandiert werden muss. Somit wird vorteilhafterweise die Trägheit der Irrigationsdruckregelung verringert, wodurch der vom Operateur gewünschte Irrigationsdruck P_{I} am Handstück 3 besonders rasch und ohne Zeitverzögerung bei einer Änderung der Stellung des Fußpedals 15 bereitgestellt wird.

Figur 4 zeigt einen oberen Abschnitt eines Anschlusselements 26 einer erfindungsgemäßen Vorrichtung, das anstatt des Anschlusselements 9 auch in der Vorrichtung 1 verwendet werden kann. Das Anschlusselement 26 umfasst einen Dorn 27 mit einem Dreiwegehahn 28, wobei an diesem eine erste Verbindung und eine zweite Verbindung einstellbar sind. In Figur 4 ist die erste Verbindung des Dreiwegehahns 28 eingestellt und zur Einstellung der zweiten Verbindung wird der Dreiwegehahn 28 um 90° im Uhrzeigersinn weiter gedreht. Im Inneren des Anschlusselements 26 erstrecken sich über den Dreiwegehahn 28 ein erster Kanal 29 und ein zweiter Kanal 30. In der ersten Verbindung des Dreiwegehahns 28 verbindet der erste Kanal 29 das Gasdruckgerät 14 mit dem Gasphasenbehältnis 10 und der zweite Kanal 30 verbindet das Gasphasenbehältnis 10 mit dem Fluidbehältnis 7. In der zweiten Verbindung des Dreiwegehahns 28 verbindet der erste Kanal 29 das Gasdruckgerät 14 mit dem Fluidbehältnis 7 und die Verbindung des zweiten Kanals 30 ist durch den Dreiwegehahn 28 unterbrochen.

Bei dem Ausführungsbeispiel einer erfindungsgemäßen Vorrichtung gemäß Figur 1 und Figur 4 wird ein erfindungsgemäßes Verfahren durchgeführt, bei dem mit dem Ablassen des Fluids 5 von dem Gasphasenbehältnis 10 in das Fluidbehältnis 7 die Operationsvorbereitung begonnen wird. Durch das Ablassen befindet sich das Fluid 5, wie für die Operation vorgesehen, im Fluidbehältnis 7 und am Dreiwegehahn 28 kann die zweite Verbindung eingestellt werden. Die dadurch eingestellte direkte Verbindung zwischen dem Gasdruckgerät 14 und dem Fluidbehältnis 7 ermöglicht, dass das Gasphasenbehältnis 10 und das Anschlusselement 26 voneinander getrennt werden. Des Weiteren kann durch Beaufschlagen des Fluidbehältnis 7 mit dem Ausgangsdruck P_{A}, am chirurgischen Handstück 3 das Fluid 5 mit dem Irrigationsdruck P_{I} bereitgestellt werden. Auch hier wird, wie bei dem Ausführungsbeispiel gemäß Figur 3, das Volumen der Gasphase 12 verringert, was vorteilhafterweise die Trägheit der Irrigationsdruckregelung senkt. Sobald die zweite Verbindung eingestellt wurde kann das Gasphasenbehältnis 10 von dem Dorn 27 abgezogen werden.

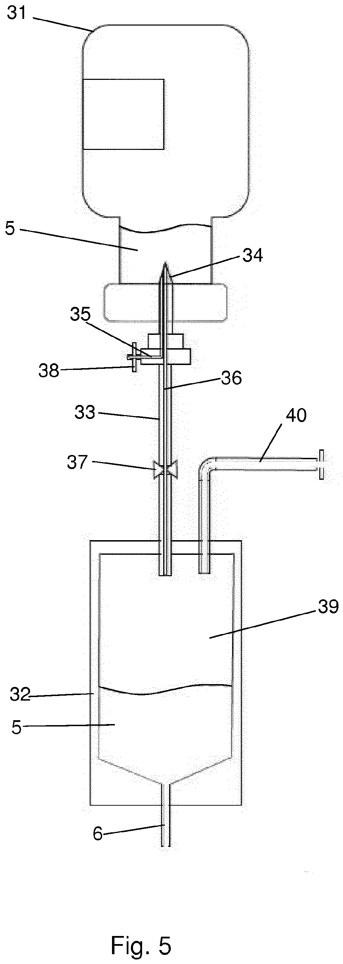

Figur 5 zeigt eine Teilansicht eines bevorzugten Ausführungsbeispiels einer erfindungsgemäßen Vorrichtung, wobei die Teilansicht den Verbindungsaufbau zwischen einem Gasphasenbehältnis 31 und einem Fluidbehältnis 32 darstellt und dieser auch in der Vorrichtung 1 einsetzbar ist. Die Darstellung zeigt einen Fluidstand während des erstens erfindungsgemäßen Verfahrensschritts. Das Gasphasenbehältnis 31 ist an einem nicht dargestellten Infusionshalter aufgehängt und über ein Anschlusselement 33 mit dem Fluidbehältnis 32, das ebenfalls am Infusionshalter aufgehängt ist, verbunden. Hierfür umfasst das Anschlusselement 33 einen Dorn 34 mit dem die Versiegelung des Gasphasenbehältnisses 31 durchbrochen wird. Zum Ablassen des Fluids 5 vom Gasphasenbehältnis 31 in das Fluidbehältnis 32 sind am Anschlusselement 33 ein erster Kanal 35 und ein zweiter Kanal 36 ausgebildet. Der zweite Kanal 36 weist ein Einwegeventil 37 auf und verbindet das Gasphasenbehältnis 31 mit dem Fluidbehältnis 32. Das Einwegeventil 37 lässt nur eine Fließrichtung von Flüssigkeiten oder Gasen zu, wobei bei diesem Ausführungsbeispiel die zulässige Fließrichtung vom Gasphasenbehältnis 31 in Richtung Fluidbehältnis 32 verläuft. Der erste Kanal 35 verbindet das Gasphasenbehältnis 31 mit dessen Umgebung, wodurch beim Ablassen des Fluids 5 in das Fluidbehältnis 32 Umgebungsluft in das Gasphasenbehältnis 31 nachströmt und ein Unterdruck im Gasphasenbehältnis 31 verhindert wird. Der erste Kanal 35 weist des Weiteren einen Filter 38 auf, der eine Verunreinigung des Fluids 5 durch Staubpartikeln aus der Umgebungsluft verhindert. Um das Fluid 5 an einem chirurgischen Handstück mit einem Irrigationsdruck P_{I} bereitzustellen, ist das chirurgische Handstück über die Leitung 6 mit dem Fluidbehältnis 32 verbunden. Zur Regelung des Irrigationsdrucks P_{I} am chirurgischen Handstück ist eine Gasphase 39 des Fluidbehältnisses 32 über eine Fluidbehältnisleitung 40, die Teil der Leitung 13 gemäß Figur 1 ist, mit einem nicht dargestellten Gasdruckgerät verbunden. Durch das Einwegeventil 37 ist nur das Fluidbehältnis 32 durch die Ausgangsdruckänderungen P_{A} des Gasdruckgeräts betroffen und das Gasphasenbehältnis kann nach Ablassen des Fluids 5 abgenommen werden, beziehungsweise die von der Druckregelung betroffene Gasphase 39 ermöglicht durch ihre auf das Fluidbehältnis 32 eingeschränkte Größe eine raschere Regelung des Irrigationsdruck P_{I}.

Es kann erwähnt werden, dass das Anschlusselement anstatt des Einwegeventils eine elastische Stelle aufweisen kann, an der der zweite Kanal durch eine Klemme versperrbar ist. Diese Klemme kann beispielsweise aus einem Klemmring, einem federbelasteten Klemmmechanismus oder anderen Mechanismen, die in der Medizin Anwendung finden, gebildet sein. Dadurch kann auch ohne Einwegeventil die von der Druckregelung betroffene Gasphase auf das Fluidbehältnis begrenzt werden. Um zu vermeiden, dass die Fluidbehältnisleitung nach Ablassen des Fluids in das Fluid ragt, weist das Fluidbehältnis ein größeres Volumen auf als das Gasphasenbehältnis.

Figur 1 und Figur 5 zeigen die Vorrichtung 1 während der Operationsvorbereitung, die im Wesentlichen aus folgenden Verfahrensschritten besteht. Das versiegelte Gasphasenbehältnis 31 ist mit dem Fluid 5 gefüllt und wird am Infusionshalter 8 aufgehängt. Bei der Operationsvorbereitung werden durch das Anschlusselement 33 das Gasphasenbehältnis 31 über den zweiten Kanal 36 mit dem Fluidbehältnis 32 verbunden und des Weiteren durch die Fluidbehältnisleitung 40 das Gasdruckgerät 14 mit dem Fluidbehältnis 32 verbunden. Erstere Verbindung wird mittels Dorn 34 hergestellt, mit dem die nicht dargestellte Versiegelung des Gasphasenbehältnisses 31 durchbrochen wird. Zur Steuerung des Irrigationsdrucks P_{I} wird gemäß dem erfindungsgemäßen Verfahren mit dem Ablassen des Fluids 5 vom Gasphasenbehältnis 31 in das Fluidbehältnis 32 begonnen. Beim Ablassen wird das Fluidbehältnis 32 über den zweiten Kanal 36 mit dem Fluid 5 gefüllt, wobei der im Gasphasenbehältnis 10 entstehende Unterdruck über den ersten gasführenden Kanal 35 ausgeglichen wird. Nach Ablassen des Fluids 5 in das Fluidbehältnis 32 ist das Gasdruckgerät 14 direkt mit der Gasphase 39 verbunden und das Fluidbehältnis 32 kann mit dem Ausgangsdruck P_{A} beaufschlagt werden. Das Einwegeventil 37 verhindert, dass der variable Ausgangsdruck P_{A} sich auch auf das Gasphasenbehältnis 31 auswirkt. Anschließend erfolgt die Bereitstellung des Fluids 5 am chirurgischen Handstück 3 mit dem Irrigationsdruck P_{I}. Durch die bidirektionale Regelung des Ausgangsdrucks P_{A} kann der Irrigationsdruck P_{I} je nach Vorgabe des Operateurs vorteilhafterweise besonders rasch erhöht oder gesenkt werden.

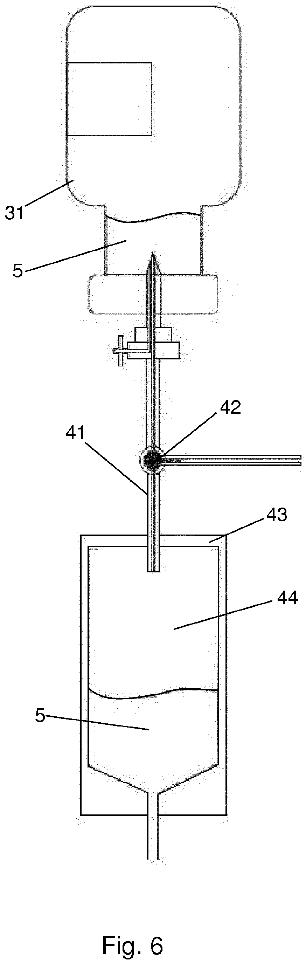

Figur 6 zeigt eine Teilansicht eines Ausführungsbeispiels einer erfindungsgemäßen Vorrichtung, das anstatt des Einwegeventils des Ausführungsbeispiels gemäß Figur 5 ein Anschlusselement 41 mit einem Dreiwegehahn 42 aufweist. Am Dreiwegehahn 42 ist eine erste Verbindung oder eine zweite Verbindung einstellbar, wobei die erste Verbindung das Gasphasenbehältnis 31 mit einem Fluidbehältnis 43 verbindet und zum Ablassen des Fluids 5 vom Gasphasenbehältnis 31 in das Fluidbehältnis 43 dient. Die zweite Verbindung verbindet ein Gasdruckgerät mit einer Gasphase 44 des Fluidbehältnisses 43 wodurch der Irrigationsdruck P_{I} eines Augenchirurgiegeräts regelbar ist und auf die Fluidbehältnisleitung gemäß Figur 5 verzichtet werden kann.

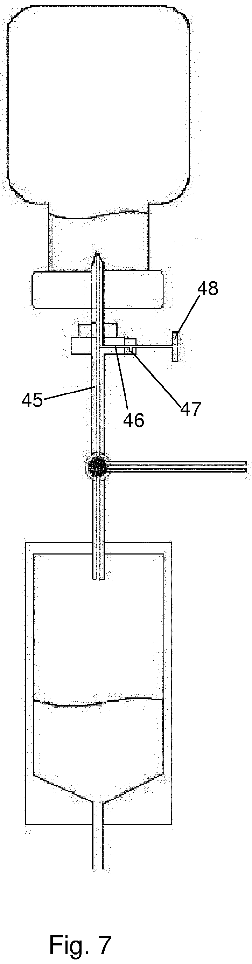

Figur 7 zeigt Teilansicht einer erfindungsgemäßen Vorrichtung die zusätzlich zum Ausführungsbeispiel gemäß Figur 6 in einem Anschlusselement 45 an einem ersten Kanal 46 eine Fluidabscheidevorrichtung 47 aufweist. Die Fluidsabscheidevorrichtung 47 verhindert, dass ein Filter 48 durch ausströmendes Fluid, durch den zweiten Kanal 46, verlegt, beziehungsweise in weitere Folge verstopft wird.

Bei den Ausführungsbeispielen der erfindungsgemäßen Vorrichtung gemäß Figur 1 und Figur 6, beziehungsweise Figur 7 entspricht das erfindungsgemäße Verfahren zur Operationsvorbereitung der Beschreibung gemäß Figur 1 und Figur 4.

Bei allen sechs vorstehend erläuterten Ausführungsbeispielen der Erfindung ist der Vorteil erhalten, dass mit bei Operationen handelsüblichen Mitteln eine sehr rasch reagierende Irrigationsdruckregelung erreicht wird, wodurch mit den Vorrichtungen eine das Auge schonende Operation durch den Operateur durchführbar ist.

## Patentansprüche

1. Vorrichtung (1) zur Verwendung in der Augenchirurgie, umfassend ein Gasdruckgerät (14) zur Bereitstellung eines variablen Ausgangsdrucks (P_{A}), ein Fluidbehältnis (7; 32; 43), zur Bereitstellung eines Fluids (5) zum Spülen eines Auges (2), wobei das Fluidbehältnis (7; 32; 43) an einer in Betriebslage unteren Seite, mit einem chirurgischen Handstück (3) verbunden ist, um das Fluid (5) mit einem vom Operateur vorgebbaren Irrigationsdruck (P_{I}) in das Auge (2) abzugeben, wobei das Fluidbehältnis (7; 32; 43) an einer in Betriebslage oberen Seite, mit einem Anschlusselement (9; 20; 26; 33; 41; 45) verbunden ist, wobei das Fluidbehältnis (7; 32; 43) über einen Dorn (16; 21; 27; 34) des Anschlusselements (9; 20; 26; 33; 41; 45), an einer unteren Seite eines Gasphasenbehältnis (10; 31), mit dem Gasphasenbehältnis (10; 31) in Verbindung steht, wobei das Gasphasenbehältnis (10, 31) zumindest zum Teil mit dem Fluid (5) befüllt ist, und wobei das Gasphasenbehältnis (10; 31) und / oder das Fluidbehältnis (7; 32; 43) eine Gasphase (12; 39; 44) aufweist und die Gasphase (12; 39; 44) über das Anschlusselement (9; 20; 26; 33; 41; 45) oder einer Fluidbehältnisleitung (40) mit dem Gasdruckgerät (14) in Verbindung steht, wodurch über den variablen Ausgangsdruck (P_{A}) des Gasdruckgeräts (14) der Irrigationsdruck (P_{I}) des chirurgischen Handstücks (3) regelbar ist und wobei im Anschlusselement (9; 20; 26; 33; 41; 45) ein erster Kanal (17; 23; 29; 35; 46) und ein zweiter Kanal (18; 24; 30; 36) ausgebildet sind, wobei über den ersten Kanal (17; 23; 29; 35; 46) das Gasphasenbehältnis (10; 31) mit dessen Umgebung oder mit dem Gasdruckgerät (14) verbunden ist, und wobei der zweite Kanal das Gasphasenbehältnis (10; 31) und das Fluidbehältnis (7; 32; 43) verbindet, **dadurch gekennzeichnet, dass** der Dorn (16; 21; 27; 34) ein Ende aufweist, welches in das Gasphasenbehältnis (10, 31) ragt, wobei während der gesamten Abgabe des Fluids mit einem vom Operateur vorgegebenen Irrigationsdrucks (P_{I}) in das Auge der erste Kanal (17; 23; 29; 35; 46) und der zweite Kanal (18; 24; 30; 36) an dem Ende des Dorns (16; 21; 27; 34) in die Gasphase (12) des Gasphasenbehältnisses (10, 31) reichen.

2. Vorrichtung gemäß einem der vorhergehenden Ansprüche, **dadurch gekennzeichnet, dass** die Verbindung zwischen dem Gasphasenbehältnis (10; 31) und dessen Umgebung oder dem Gasdruckgerät (14) eine Fluidabscheidevorrichtung (19; 47) aufweist.

3. Vorrichtung gemäß einem der vorhergehenden Ansprüche, **dadurch gekennzeichnet, dass** der zweite Kanal (36) ein Einwegeventil (37) aufweist.

4. Vorrichtung gemäß den Ansprüchen 1 bis 2, **dadurch gekennzeichnet, dass** das Anschlusselement eine elastische Stelle aufweist, an der der zweite Kanal durch eine Klemme versperrbar ist.

5. Vorrichtung gemäß den Ansprüchen 1 bis 2, **dadurch gekennzeichnet, dass** das Anschlusselement (22) von dem Gasphasenbehältnis (10) trennbar ausgebildet ist und, dass Abdeckmittel (22) vorgesehen sind, die durch Abdecken des Dorns (21) zum gasdichten Verbinden des ersten Kanals (23) mit dem zweiten Kanal (24) des Anschlusselements (20) ausgebildet ist.

6. Vorrichtung gemäß den Ansprüchen 1 bis 2, **dadurch gekennzeichnet, dass** das Anschlusselement (26) einen Dreiwegehahn (28) aufweist, wodurch eine erste oder eine zweite Verbindung einstellbar ist, wobei zwischen dem Gasdruckgerät (14), über den Dreiwegehahn (28), dem Gasphasenbehältnis (10) und dem Fluidbehältnis (7) die erste Verbindung besteht und wobei die zweite Verbindung zwischen dem Gasdruckgerät (14) über den Dreiwegehahn (28) und dem Fluidbehältnis (7) besteht.

7. Vorrichtung gemäß den Ansprüchen 1 bis 2, **dadurch gekennzeichnet, dass** das Anschlusselement (41; 45) einen Dreiwegehahn (42) aufweist, wodurch eine erste oder eine zweite Verbindung einstellbar ist, wobei die erste Verbindung ein Gasphasenbehältnis (31) mit dem Fluidbehältnis (43) verbindet, und wobei die zweite Verbindung ein Gasdruckgerät mit einem Fluidbehältnis (43) verbindet.

8. Vorrichtung gemäß einem der Ansprüche 1 bis 7, **dadurch gekennzeichnet, dass** das Anschlusselement einen Dorn eines handelsüblichen Infusionsbestecks aufweist.

9. Vorrichtung gemäß einem der vorhergehenden Ansprüche, **dadurch gekennzeichnet, dass** das Fluidbehältnis (7; 32; 43) durch einen Infusionsbeutel gebildet ist.

10. Vorrichtung gemäß einem der vorhergehenden Ansprüche, **dadurch gekennzeichnet, dass** das Gasphasenbehältnis (10; 31) durch eine handelsübliche Flasche, die mit einer Kochsalzlösung gefüllt ist, gebildet ist.

11. Verfahren zum Bereitstellen des Fluids (5) am chirurgischen Handstücks (3) an einer Vorrichtung (1) gemäß einem der vorhergehenden Ansprüche, wobei folgende Verfahrensschritte durch geführt werden:
• Ablassen des im Wesentlichen gesamten Fluids (5) vom Gasphasenbehältnis (10; 31) in das Fluidbehältnis (7; 32; 43);
• Beaufschlagen des Fluidbehältnisses (7; 32; 43) mit Ausgangsdruck (P_{A});
• Bereitstellen des Fluids (5) am chirurgischen Handstück (3) mit dem vom Operateur regelbaren Irrigationsdruck (P_{I}).

12. Verfahren gemäß Anspruch 11, **dadurch gekennzeichnet, dass** bevor das Fluidbehältnis (7) mit dem Ausgangsdruck (P_{A}) beaufschlagt wird, das Gasphasenbehältnis (10) vom Anschlusselement (20) getrennt und das Anschlusselement (20) mit dem Abdeckmittel (22) abgedeckt wird.

13. Verfahren gemäß Anspruch 11, **dadurch gekennzeichnet, dass** bevor das Fluidbehältnis (7, 43) mit dem Ausgangsdruck P_{A} beaufschlagt wird, am Dreiwegehahn (28; 42) die zweite Verbindung eingestellt und das Gasphasenbehältnis (10; 31) vom Anschlusselement (26; 41; 45) getrennt wird.

## Claims

1. A device (1) for use in eye surgery comprising a gas pressure device (14) for providing a variable initial pressure (P_{A}), a fluid container (7; 32; 43) for providing a fluid (5) for rinsing an eye (2), wherein the fluid container (7; 32; 43) is connected to a surgical handpiece (3) on a side which is lower in the operating position in order to deliver the fluid (5) into the eye (2) at an irrigation pressure (P_{I}) predeterminable by the surgeon, wherein the fluid container (7; 32; 43) is connected to a connecting element (9; 20; 26; 33; 41; 45) on a side which is upper in the operating position, wherein the fluid container (7; 32; 43) communicates with a gas phase container (10; 31) via a mandrel (16; 21; 27; 34) of the connecting element (9; 20; 26; 33; 41; 45) on a lower side of the gas phase container (10; 31), with the gas phase container (10; 31) being filled at least partially with the fluid (5), and wherein the gas phase container (10; 31) and/or the fluid container (7; 32; 43) exhibit(s) a gas phase (12; 39; 44) and the gas phase (12; 39; 44) communicates with the gas pressure device (14) via the connecting element (9; 20; 26; 33; 41; 45) or a fluid container line (40), whereby the irrigation pressure (P_{I}) of the surgical handpiece (3) is adjustable via the variable initial pressure (P_{A}) of the gas pressure device (14), and wherein a first channel (17; 23; 29; 35; 46) and a second channel (18; 24; 30; 36) are formed in the connecting element (9; 20; 26; 33; 41; 45), wherein the gas phase container (10; 31) is connected with the surroundings thereof or to the gas pressure device (14) via the first channel (17; 23; 29; 35; 46) and wherein the second channel connects the gas phase container (10; 31) and the fluid container (7; 32; 43), **characterized in that** the mandrel (16; 21; 27; 34) has an end projecting into the gas phase container (10; 31), wherein the first channel (17; 23; 29; 35; 46) and the second channel (18; 24; 30; 36), at the end of the mandrel (16; 21; 27; 34), extend into the gas phase (12) of the gas phase container (10; 31) during the entire delivery of the fluid into the eye at an irrigation pressure (P_{I}) predetermined by the surgeon.

2. A device according to any of the preceding claims, **characterized in that** the connection between the gas phase container (10; 31) and the surroundings thereof or the gas pressure device (14) exhibits a fluid deposition device (19; 47).

3. A device according to any of the preceding claims, **characterized in that** the second channel (36) exhibits a one-way valve (37).

4. A device according to claims 1 to 2, **characterized in that** the connecting element exhibits an elastic spot in which the second channel is lockable by a clamp.

5. A device according to claims 1 to 2, **characterized in that** the connecting element (22) is designed so as to be separable from the gas phase container (10) and that a covering means (22) is provided which is designed by covering the mandrel (21) in order to connect the first channel (23) to the second channel (24) of the connecting element (20) in a gastight manner.

6. A device according to claims 1 to 2, **characterized in that** the connecting element (26) comprises a three-way stopcock (28), whereby a first or a second connection is adjustable, wherein the first connection exists between the gas pressure device (14) via the three-way stopcock (28), the gas phase container (10) and the fluid container (7) and wherein the second connection exists between the gas pressure device (14) via the three-way stopcock (28) and the fluid container (7).

7. A device according to claims 1 to 2, **characterized in that** the connecting element (41; 45) comprises a three-way stopcock (42), whereby a first or a second connection is adjustable, wherein the first connection connects a gas phase container (31) to the fluid container (43) and wherein the second connection connects a gas pressure device to a fluid container (43).

8. A device according to any of claims 1 to 7, **characterized in that** the connecting element comprises a mandrel of a commercially available infusion set.

9. A device according to any of the preceding claims, **characterized in that** the fluid container (7; 32; 43) is configured by an infusion bag.

10. A device according to any of the preceding claims, **characterized in that** the gas phase container (10; 31) is configured by a commercially available bottle filled with a saline solution.

11. A method of providing the fluid (5) at the surgical handpiece (3) on a device (1) according to any of the preceding claims, wherein the following procedural steps are performed:
• draining essentially the entire fluid (5) from the gas phase container (10; 31) into the fluid container (7; 32; 43);
• charging the fluid container (7; 32; 43) with the initial pressure (P_{A});
• providing the fluid (5) at the surgical handpiece (3) at the irrigation pressure (P_{I}) adjustable by the surgeon.

12. A method according to claim 11, **characterized in that** the gas phase container (10) is separated from the connecting element (20) and the connecting element (20) is covered with the covering means (22) before the fluid container (7) is charged with the initial pressure (P_{A}).

13. A method according to claim 11, **characterized in that** the second connection is set on the three-way stopcock (28; 42) and the gas phase container (10; 31) is separated from the connecting element (26; 41; 45) before the fluid container (7, 43) is charged with the initial pressure (P_{A}).

## Revendications

1. Dispositif (1) destiné à être utilisé en chirurgie oculaire, comprenant un appareil pneumatique (14) pour la fourniture d'une pression de sortie (P_{A}) variable, un récipient à fluide (7 ; 32 ; 43) pour la fourniture d'un fluide (5) de rinçage d'un oeil (2), où le récipient à fluide (7 ; 32 ; 43) est relié à une pièce à main chirurgicale (3) qui lui est inférieure en position de fonctionnement pour administrer le fluide (5) dans l'oeil (2) avec une pression d'irrigation (P_{I}) pouvant être définie par l'opérateur, où le récipient à fluide (7 ; 32 ; 43) est relié à un élément de connexion (9 ; 20 ; 26 ; 33 ; 41 ; 45) qui lui est supérieur en position de fonctionnement, où le récipient à fluide (7 ; 32 ; 43) est relié à un récipient à phase vapeur (10 ; 31) par un mandrin (16 ; 21 ; 27 ; 34) de l'élément de connexion (9 ; 20 ; 26 ; 33 ; 41 ; 45) sur le bas du récipient à phase vapeur (10 ; 31), où ledit récipient à phase vapeur (10, 31) est au moins partiellement rempli de fluide (5), et où le récipient à phase vapeur (10 ; 31) et/ou le récipient à fluide (7 ; 32 ; 43) comprend une phase gazeuse (12 ; 39 ; 44) et la phase gazeuse (12 ; 39 ; 44) est reliée à l'appareil pneumatique (14) par l'intermédiaire de l'élément de connexion (9 ; 20 ; 26 ; 33 ; 41 ; 45) ou d'un conduit (40) du récipient à fluide, de sorte que la pression d'irrigation (P_{I}) de la pièce à main chirurgicale (3) peut être réglée au moyen de la pression de sortie (P_{A}) variable de l'appareil pneumatique (14), et où un premier canal (17 ; 23 ; 29 ; 35 ; 46) et un deuxième canal (18 ; 24 ; 30 ; 36) sont prévus dans l'élément de connexion (9 ; 20 ; 26 ; 33 ; 41 ; 45), le récipient à phase vapeur (10 ; 31) étant relié à son environnement ou à l'appareil pneumatique (14) par le premier canal (17 ; 23 ; 29 ; 35 ; 46), et le deuxième canal reliant le récipient à phase vapeur (10 ; 31) et le récipient à fluide (7 ; 32 ; 43), **caractérisé en ce que** le mandrin (16 ; 21 ; 27 ; 34) présente une extrémité pénétrant dans le récipient à phase vapeur (10, 31), le premier canal (17 ; 23 ; 29 ; 35 ; 46) et le deuxième canal (18 ; 24 ; 30 ; 36) atteignant à l'extrémité du mandrin (16 ; 21 ; 27 ; 34) la phase gazeuse (12) du récipient à phase vapeur (10, 31) pendant toute la durée d'administration du fluide dans l'oeil avec une pression d'irrigation (P_{I}) à définir par l'opérateur.

2. Dispositif selon l'une des revendications précédentes, **caractérisé en ce que** la liaison entre le récipient à phase vapeur (10 ; 31) et son environnement ou l'appareil pneumatique (14) comporte un dispositif séparateur de fluide (19 ; 47).

3. Dispositif selon l'une des revendications précédentes, **caractérisé en ce que** le deuxième canal (36) comporte une vanne unidirectionnelle (37).

4. Dispositif selon les revendications 1 et 2, **caractérisé en ce que** l'élément de connexion présente un emplacement élastique où le deuxième canal peut être obturé par une pince.

5. Dispositif selon les revendications 1 et 2, **caractérisé en ce que** l'élément de connexion (22) est prévu séparable du récipient à phase vapeur (10) et **en ce qu'**un moyen de couverture (22) est prévu pour le raccord étanche aux gaz du premier canal (23) avec le deuxième canal (24) de l'élément de connexion (20) par couverture du mandrin (21).

6. Dispositif selon les revendications 1 et 2, **caractérisé en ce que** l'élément de connexion (26) comporte une vanne trois voies (28) permettant de réaliser une première ou une deuxième connexion, la première connexion étant réalisée par la vanne trois voies (28) entre l'appareil pneumatique (14), le récipient à phase vapeur (10) et le récipient à fluide (7), et la deuxième connexion par la vanne trois voies (28) entre l'appareil pneumatique (14) et le récipient à fluide (7).

7. Dispositif selon les revendications 1 et 2, **caractérisé en ce que** l'élément de connexion (41 ; 45) comporte une vanne trois voies (42) permettant de réaliser une première ou une deuxième connexion, la première connexion reliant un récipient à phase vapeur (31) au récipient à fluide (43), et la deuxième connexion reliant un appareil pneumatique à un récipient à fluide (43).

8. Dispositif selon l'une des revendications 1 à 7, **caractérisé en ce que** l'élément de connexion comporte un mandrin d'un dispositif de perfusion du commerce.

9. Dispositif selon l'une des revendications précédentes, **caractérisé en ce que** le récipient à fluide (7 ; 32 ; 43) est formé par une poche pour perfusion.

10. Dispositif selon l'une des revendications précédentes, **caractérisé en ce que** le récipient à phase vapeur (10 ; 31) est formé par une bouteille du commerce remplie d'une solution saline.

11. Procédé de fourniture du fluide (5) à la pièce à main chirurgicale (3) d'un dispositif (1) selon l'une des revendications précédentes, où sont exécutées les étapes de procédé suivantes :
• vidange de presque tout le fluide (5) du récipient à phase vapeur (10 ; 31) vers le récipient à fluide (7 ; 32 ; 43) ;
• application d'une pression de sortie (P_{A}) sur le récipient à fluide (7 ; 32 ; 43) ;
• refoulement du fluide (5) sur la pièce à main chirurgicale (3) par pression d'irrigation (P_{I}) réglable par l'opérateur.

12. Procédé selon la revendication 11, **caractérisé en ce que** préalablement à l'application de la pression de sortie (P_{A}) sur le récipient à fluide (7), le récipient à phase vapeur (10) est séparé de l'élément de connexion (20) et l'élément de connexion (20) est recouvert du moyen de couverture (22).

13. Procédé selon la revendication 11, **caractérisé en ce que** préalablement à l'application de la pression de sortie (P_{A}) sur le récipient à fluide (7, 43), la deuxième connexion est réglée sur la vanne trois voies (28 ; 42) et le récipient à phase vapeur (10 ; 31) est séparé de l'élément de connexion (26 ; 41 ; 45).
